# EUROPEAN PATENT SPECIFICATION

(11) **EP 0 399 666 B1**
(45) Date of publication and mention of the grant of the patent: **28.07.1993**
(21) Application number: 90304575.5
(22) Date of filing: 26.04.1990
(51) Int. Cl.: C12N 15/62, C07K 13/00, C12P 21/02

(54) **Fusion proteins containing N-terminal fragments of human serum albumin**
N-terminale Fragmente von menschliches Serumalbumin enthaltenden Fusionsproteinen
Protéines de fusion contenant des fragments N-terminaux de l'albumine de sérum humaine

(30) Priority: 29.04.1989 GB 8909919
(43) Date of publication of application: 28.11.1990
(73) Proprietor: Delta Biotechnology Limited, Nottingham NG7 1FD (GB)
(72) Inventor: Ballance, David James, West Bridgford, Nottingham NG2 7AG (GB)
(74) Representative: Bassett, Richard Simon

(56) References cited:
- EP-A- 0 308 381
- EP-A- 0 322 094

## Description

The present invention relates to fusion polypeptides where two individual polypeptides or parts thereof are fused to form a single amino acid chain. Such fusion may arise from the expression of a single continuous coding sequence formed by recombinant DNA techniques.

Fusion polypeptides are known, for example those where a polypeptide which is the ultimately desired product of the process is expressed with an N-terminal "leader sequence" which encourages or allows secretion of the polypeptide from the cell. An example is disclosed in EP-A-116 201 (Chiron).

Human serum albumin (HSA) is a known protein found in the blood. EP-A-147 198 (Delta Biotechnology) discloses its expression in a transformed host, in this case yeast. Our earlier application EP-A-322 094 discloses N-terminal fragments of HSA, namely those consisting of residues 1-n where n is 369 to 419, which have therapeutic utility. The application also mentions the possibility of fusing the C-terminal residue of such molecules to other, unnamed, polypeptides.

One aspect of the present invention provides a fusion polypeptide comprising, as at least part of the N-terminal portion thereof, an N-terminal portion of HSA or a variant thereof and, as at least part of the C-terminal portion thereof, another polypeptide except that, when the said N-terminal portion of HSA is the 1-n portion where n is 369 to 419 or a variant thereof then the said polypeptide is (a) the 585 to 1578 portion of human fibronectin or a variant thereof, (b) the 1 to 368 portion of CD4 or a variant thereof, (c) platelet derived growth factor, or a variant thereof, (d) transforming growth factor, or a variant thereof, (e) the 1-261 portion of mature human plasma fibronectin or a variant thereof, (f) the 278-578 portion of mature human plasma fibronectin or a variant thereof, (g) the 1-272 portion of mature human von Willebrand's Factor or a variant thereof, or (h) alpha-1-antitrypsin or a variant thereof.

The N-terminal portion of HSA is preferably the said 1-n portion, the 1-177 portion (up to and including the cysteine), the 1-200 portion (up to but excluding the cysteine) or a portion intermediate 1-177 and 1-200.

The term "human serum albumin" (HSA) is intended to include (but not necessarily to be restricted to) known or yet-to-be-discovered polymorphic forms of HSA. For example, albumin Naskapi has Lys-372 in place of Glu-372 and pro-albumin Christchurch has an altered pro-sequence. The term "variants" is intended to include (but not necessarily to be restricted to) minor artificial variations in sequence (such as molecules lacking one or a few residues, having conservative substitutions or minor insertions of residues, or having minor variations of amino acid structure). Thus polypeptides which have 80%, preferably 85%, 90%, 95% or 99%, homology with HSA are deemed to be "variants". It is also preferred for such variants to be physiologically equivalent to HSA; that is to say, variants preferably share at least one pharmacological utility with HSA. Furthermore, any putative variant which is to be used pharmacologically should be non-immunogenic in the animal (especially human) being treated.

Conservative substitutions are those where one or more amino acids are substituted for others having similar properties such that one skilled in the art of polypeptide chemistry would expect at least the secondary structure, and preferably the tertiary structure, of the polypeptide to be substantially unchanged. For example, typical such substitutions include asparagine for glutamine, serine for asparagine and arginine for lysine. Variants may alternatively, or as well, lack up to ten (preferably only one or two) intermediate amino acid residues (ie not at the termini of the said N-terminal portion of HSA) in comparison with the corresponding portion of natural HSA; preferably any such omissions occur in the 100 to 369 portion of the molecule (relative to mature HSA itself) (if present). Similarly, up to ten, but preferably only one or two, amino acids may be added, again in the 100 to 369 portion for preference (if present). The term "physiologically functional equivalents" also encompasses larger molecules comprising the said sequence plus a further sequence at the N-terminal (for example, pro-HSA, pre-pro-HSA and met-HSA).

Clearly, the said "another polypeptide" in the fusion compounds of the invention cannot be the remaining portion of HSA, since otherwise the whole polypeptide would be HSA, which would not then be a "fusion polypeptide".

Even when the HSA-like portion is not the said 1-n portion of HSA, it is preferred for the non-HSA portion to be one of the said (a) to (h) entities.

The 1 to 368 portion of CD4 represents the first four disulphide-linked immunoglobulin-like domains of the human T lymphocyte CD4 protein, the gene for and amino acid sequence of which are disclosed in D. Smith et al (1987) Science 328, 1704-1707. It is used to combat HIV infections.

The sequence of human platelet-derived growth factor (PDGF) is described in Collins et al (1985) Nature 316, 748-750. Similarly, the sequence of transforming growth factors β (TGF-β) is described in Derynck et al (1985) Nature 316, 701-705. These growth factors are useful for wound-healing.

A cDNA sequence for the 1-261 portion of Fn was disclosed in EP-A-207 751 (obtained from plasmid pFH6 with endonuclease PvuII). This portion binds fibrin and can be used to direct fused compounds to blood clots.

A cDNA sequence for the 278-578 portion of Fn, which contains a collagen-binding domain, was disclosed by R.J. Owens and F.E. Baralle in 1986 E.M.B.O.J. 5, 2825-2830. This portion will bind to platelets.

The 1-272 portion of von Willebrand's Factor binds and stabilises factor VIII. The sequence is given in Bontham et al, Nucl. Acids Res. 14, 7125-7127.

Variants of alpha-1-antitrypsin include those disclosed by Rosenburg et al (1984) Nature 312, 77-80. In particular, the present invention includes the Pittsburgh variant (Met³⁵⁸ is mutated to Arg) and the variant where Pro³⁵⁷ and Met³⁵⁸ are mutated to alanine and arginine respectively. These compounds are useful in the treatment of septic shock and lung disorders.

Variants of the non-HSA portion of the polypeptides of the invention include variations as discussed above in relation to the HSA portion, including those with conservative amino acid substitutions, and also homologues from other species.

The fusion polypeptides of the invention may have N-terminal amino acids which extend beyond the portion corresponding to the N-terminal portion of HSA. For example, if the HSA-like portion corresponds to an N-terminal portion of mature HSA, then pre-, pro-, or pre-pro sequences may be added thereto, for example the yeast alpha-factor leader sequence. The fused leader portions of WO 90/01063 may be used. The polypeptide which is fused to the HSA portion may be a naturally-occurring polypeptide, a fragment thereof or a novel polypeptide, including a fusion polypeptide. For example, in Example 3 below, a fragment of fibronectin is fused to the HSA portion via a 4 amino acid linker.

It has been found that the amino terminal portion of the HSA molecule is so structured as to favour particularly efficient translocation and export of the fusion compounds of the invention in eukaryotic cells.

A second aspect of the invention provides a transformed host having a nucleotide sequence so arranged as to express a fusion polypeptide as described above. By "so arranged", we mean, for example, that the nucleotide sequence is in correct reading frame with an appropriate RNA polymerase binding site and translation start sequence and is under the control of a suitable promoter. The promoter may be homologous with or heterologous to the host. Downstream (3') regulatory sequences may be included if desired, as is known. The host is preferably yeast (for example Saccharomyces spp., e.g. S. cerevisiae; Kluyveromyces spp., e.g. K. lactis; Pichia spp.; or Schizosaccharomyces spp., e.g. S. pombe) but may be any other suitable host such as E. coli, B. subtilis, Aspergillus spp., mammalian cells, plant cells or insect cells.

A third aspect of the invention provides a process for preparing a fusion polypeptide according to the first aspect of the invention by cultivation of a transformed host according to the second aspect of the invention, followed by separation of the fusion polypeptide in a useful form.

A fourth aspect of the invention provides therapeutic methods of treatment of the human or other animal body comprising administration of such a fusion polypeptide.

In the methods of the invention we are particularly concerned to improve the efficiency of secretion of useful therapeutic human proteins from yeast and have conceived the idea of fusing to amino-terminal portions of HSA those proteins which may ordinarily be only inefficiently secreted. One such protein is a potentially valuable wound-healing polypeptide representing amino acids 585 to 1578 of human fibronectin (referred to herein as Fn 585-1578). As we have described in a separate application (filed simultaneously herewith) this molecule contains cell spreading, chemotactic and chemokinetic activities useful in healing wounds. The fusion polypeptides of the present invention wherein the C-terminal portion is Fn 585-1578 can be used for wound healing applications as biosynthesised, especially where the hybrid human protein will be topically applied. However, the portion representing amino acids 585 to 1578 of human fibronectin can if desired be recovered from the fusion protein by preceding the first amino acid of the fibronectin portion by amino acids comprising a factor X cleavage site. After isolation of the fusion protein from culture supernatant, the desired molecule is released by factor X cleavage and purified by suitable chromatography (e.g. ion-exchange chromatography). Other sites providing for enzymatic or chemical cleavage can be provided, either by appropriate juxtaposition of the N-terminal and C-terminal portions or by the insertion therebetween of an appropriate linker.

At least some of the fusion polypeptides of the invention, especially those including the said CD4 and vWF fragments, PDGF and α₁AT, also have an increased half-life in the blood and therefore have advantages and therapeutic utilities themselves, namely the therapeutic utility of the non-HSA portion of the molecule. In the case of α₁AT and others, the compound will normally be administered as a one-off dose or only a few doses over a short period, rather than over a long period, and therefore the compounds are less likely to cause an immune response.

### EXAMPLES : SUMMARY

Standard recombinant DNA procedures were as described by Maniatis et al (1982 and recent 2nd edition) unless otherwise stated. Construction and analysis of phage M13 recombinant clones was as described by Messing (1983) and Sanger et al (1977).

DNA sequences encoding portions of human serum albumin used in the construction of the following molecules are derived from the plasmids mHOB12 and pDBD2 (EP-A-322 094, Delta Biotechnology Ltd, relevant portions of which are reproduced below) or by synthesis of oligonucleotides equivalent to parts of this sequence. DNA sequences encoding portions of human fibronectin are derived from the plasmid pFHDEL1, or by synthesis of oligonucleotides equivalent to parts of this sequence. Plasmid pFHDEL1, which contains the complete human cDNA encoding plasma fibronectin, was obtained by ligation of DNA derived from plasmids pFH6, 16, 54, 154 and 1 (EP-A-207 751; Delta Biotechnology Ltd).

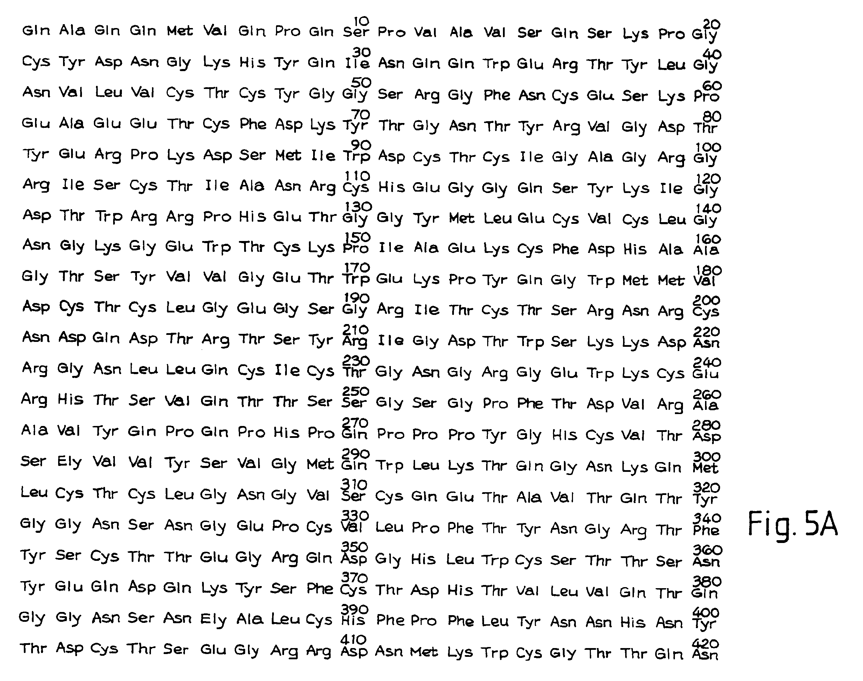

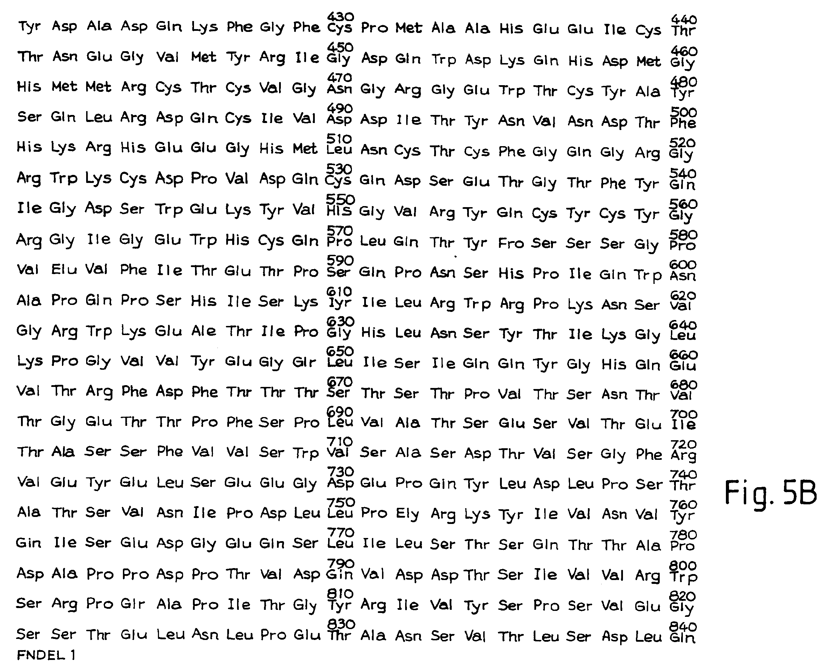

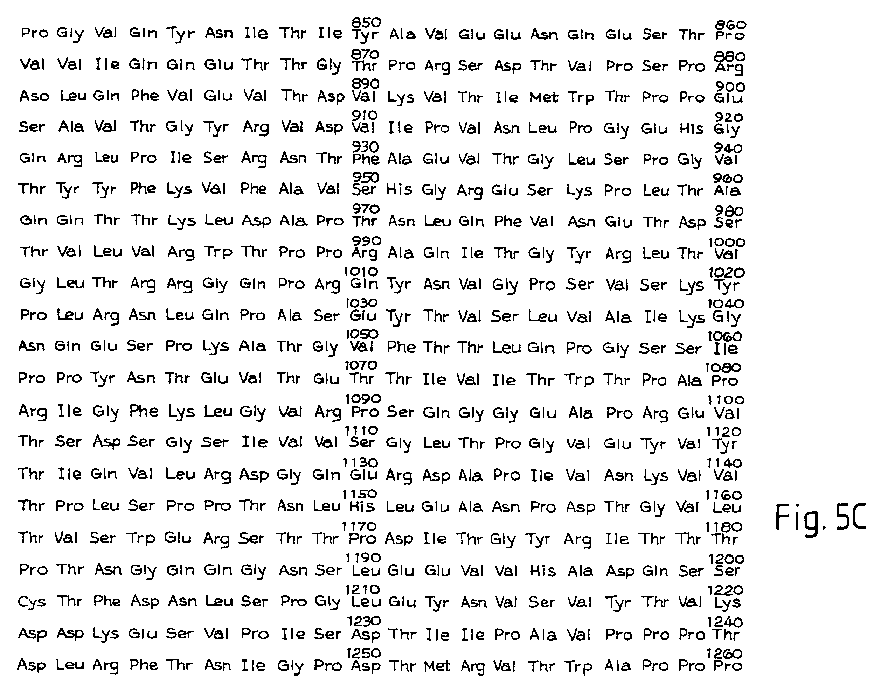

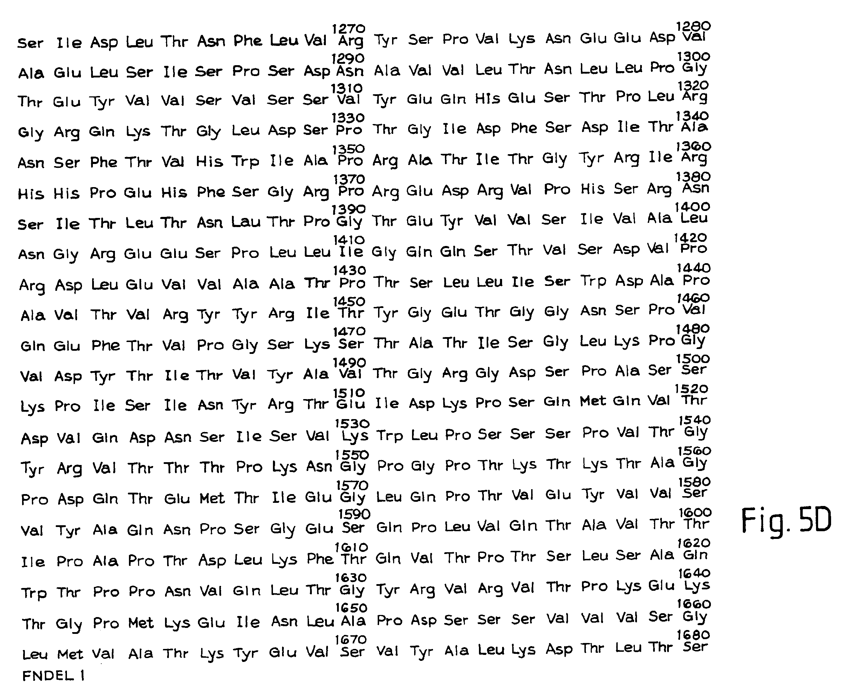

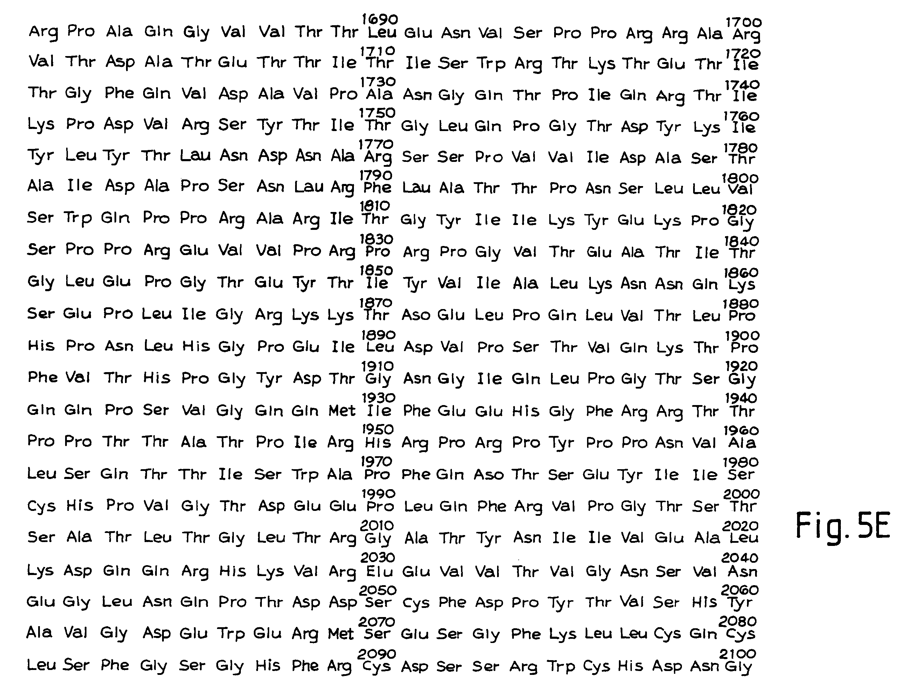

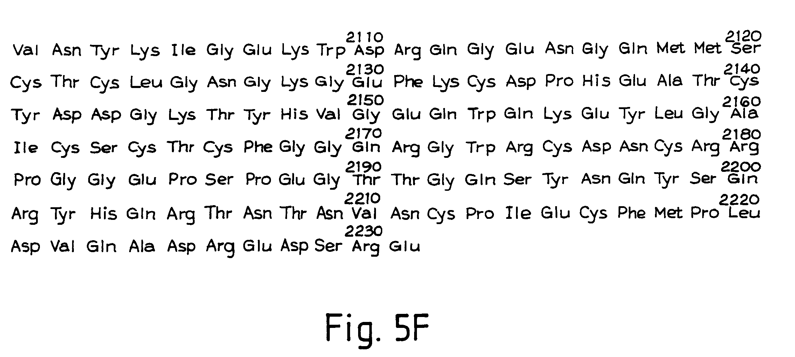

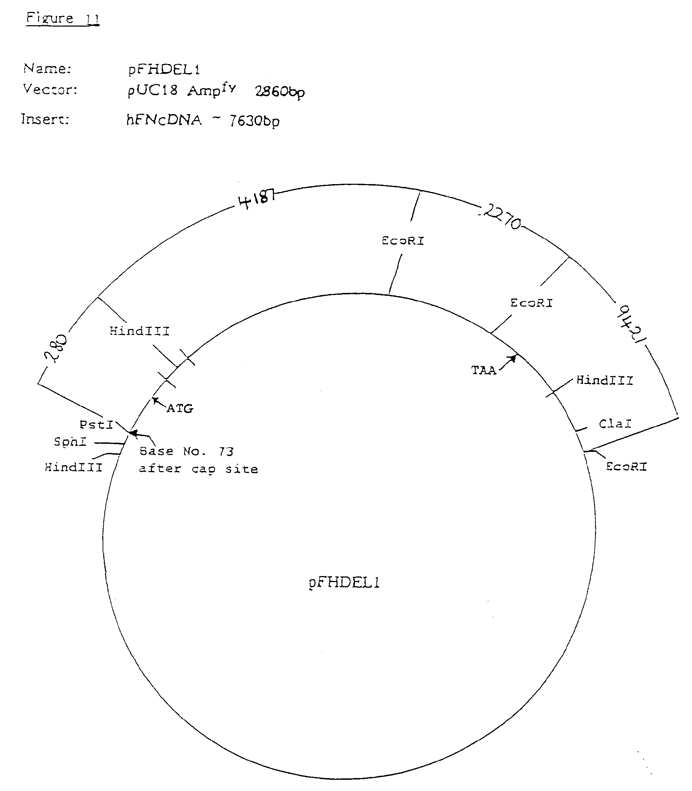

This DNA represents an mRNA variant which does not contain the 'ED' sequence and had an 89-amino acid variant of the III-CS region (R.J. Owens, A.R. Kornblihtt and F.E. Baralle (1986) Oxford Surveys on Eukaryotic Genes 3 141-160). The map of this vector is disclosed in Fig. 11 and the protein sequence of the mature polypeptide produced by expression of this cDNA is shown in Fig. 5.

Oligonucleotides were synthesised on an Applied Biosystems 380B oligonucleotide synthesiser according to the manufacturer's recommendations (Applied Biosystems, Warrington, Cheshire, UK).

An expression vector was constructed in which DNA encoding the HSA secretion signal and mature HSA up to and including the 387th amino acid, leucine, fused in frame to DNA encoding a segment of human fibronectin representing amino acids 585 to 1578 inclusive, was placed downstream of the hybrid promoter of EP-A-258 067 (Delta Biotechnology), which is a highly efficient galactose-inducible promoter functional in Saccharomyces cerevisiae. The codon for the 1578th amino acid of human fibronectin was directly followed by a stop codon (TAA) and then the S. cerevisiae phosphoglycerate kinase (PGK) gene transcription terminator. This vector was then introduced into S. cerevisiae by transformation, wherein it directed the expression and secretion from the cells of a hybrid molecule representing the N-terminal 387 amino acids of HSA C-terminally fused to amino acids 585 to 1578 of human fibronectin.

In a second example a similar vector is constructed so as to enable secretion by S. cerevisiae of a hybrid molecule representing the N-terminal 195 amino acids of HSA C-terminally fused to amino acids 585 to 1578 of human fibronectin.

Aspects of the present invention will now be described by way of example and with reference to the accompanying drawings, in which:

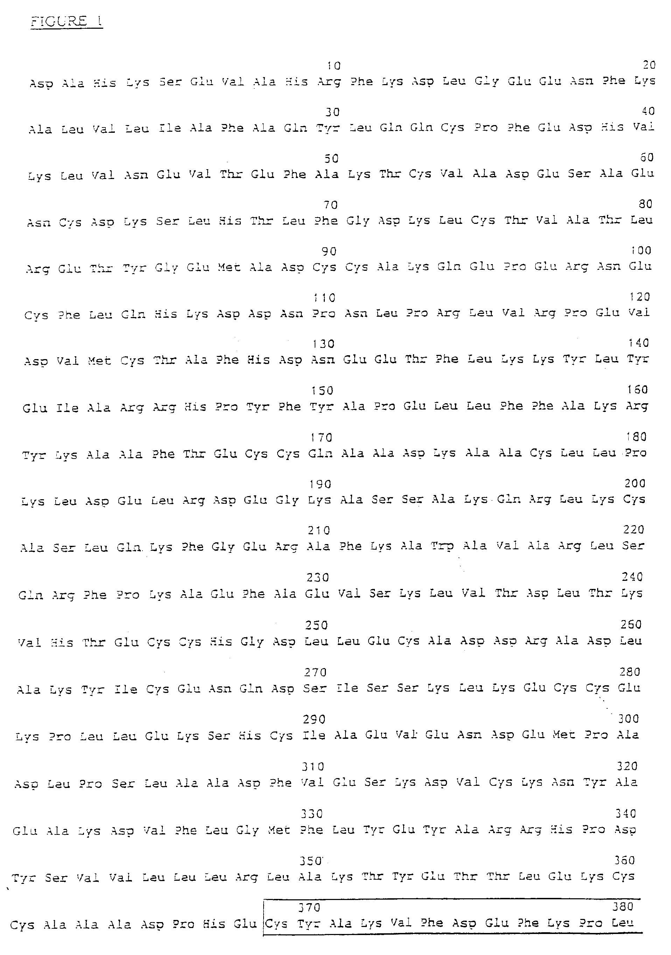

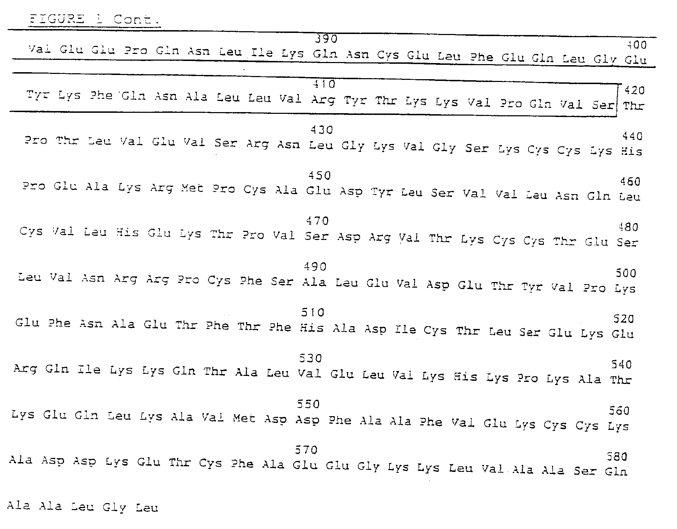

Figure 1 (on two sheets) depicts the amino acid sequence currently thought to be the most representative of natural HSA, with (boxed) the alternative C-termini of HSA(1-n);
Figure 2 (on two sheets) depicts the DNA sequence coding for mature HSA, wherein the sequence included in Linker 3 is underlined;
Figure 3 illustrates, diagrammatically, the construction of mHOB16;
Figure 4 illustrates, diagrammatically, the construction of pHOB31;
Figure 5 (on 6 sheets) illustrates the mature protein sequence encoded by the Fn plasmid pFHDEL1;
Figure 6 illustrates Linker 5, showing the eight constituent oligonucleotides;
Figure 7 shows schematically the construction of plasmid pDBDF2;
Figure 8 shows schematically the construction of plasmid pDBDF5;
Figure 9 shows schematically the construction of plasmid pDBDF9;
Figure 10 shows schematically the construction of plasmid DBDF12, using plasmid pFHDEL1; and
Figure 11 shows a map of plasmid pFHDEL1.

### EXAMPLE 1 : HSA 1-387 FUSED TO Fn 585-1578

The following is an account of a preparation of plasmids comprising sequences encoding a portion of HSA, as is disclosed in EP-A-322 094.

The human serum albumin coding sequence used in the construction of the following molecules is derived from the plasmid M13mp19.7 (EP-A-201 239, Delta Biotech- nology Ltd.) or by synthesis of oligonucleotides equivalent to parts of this sequence. Oligonucleotides were synthesised using phosphoramidite chemistry on an Applied Biosystems 380B oligonucleotide synthesizer according to the manufacturer's recommendations (AB Inc., Warrington, Cheshire, England).

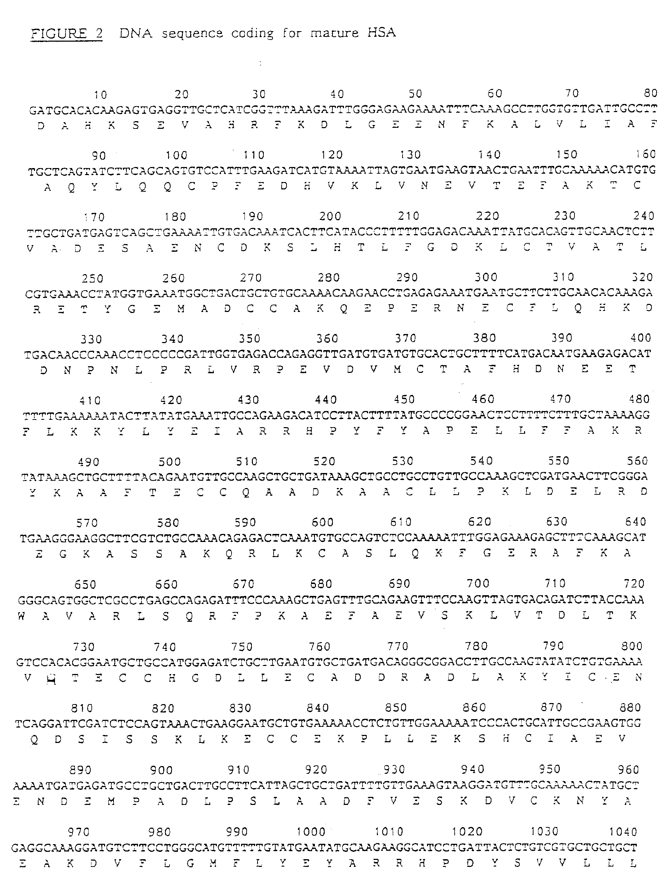

An oligonucleotide was synthesised (Linker A) which represented a part of the known HSA coding sequence (Figure 2) from the PstI site (1235-1240, Figure 2) to the codon for valine 381 wherein that codon was changed from GTG to GTC:

### Linker 1

D P H E C Y
5′ GAT CCT CAT GAA TGC TAT
3′ ACGT CTA GGA GTA CTT ACG ATA
1247

A K V F D E F K
GCC AAA GTG TTC GAT GAA TTT AAA
CGG TTT CAC AAG CTA CTT AAA TTT
1267
P L V
CTT GTC 3′
GGA CAG 5′

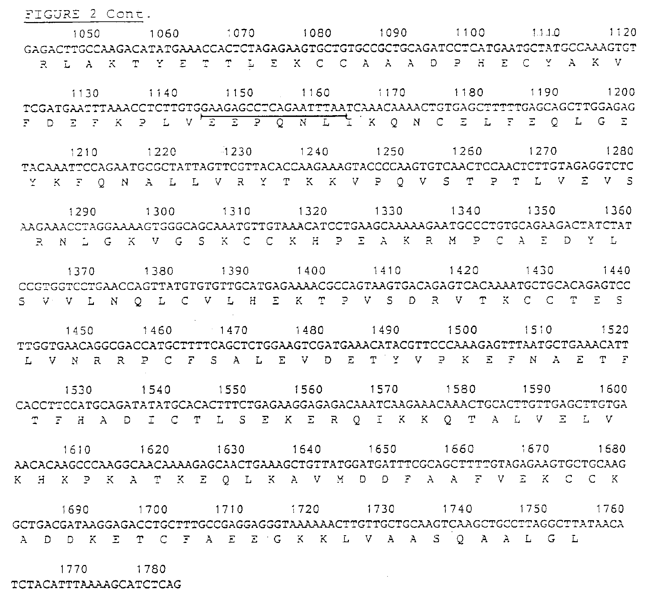

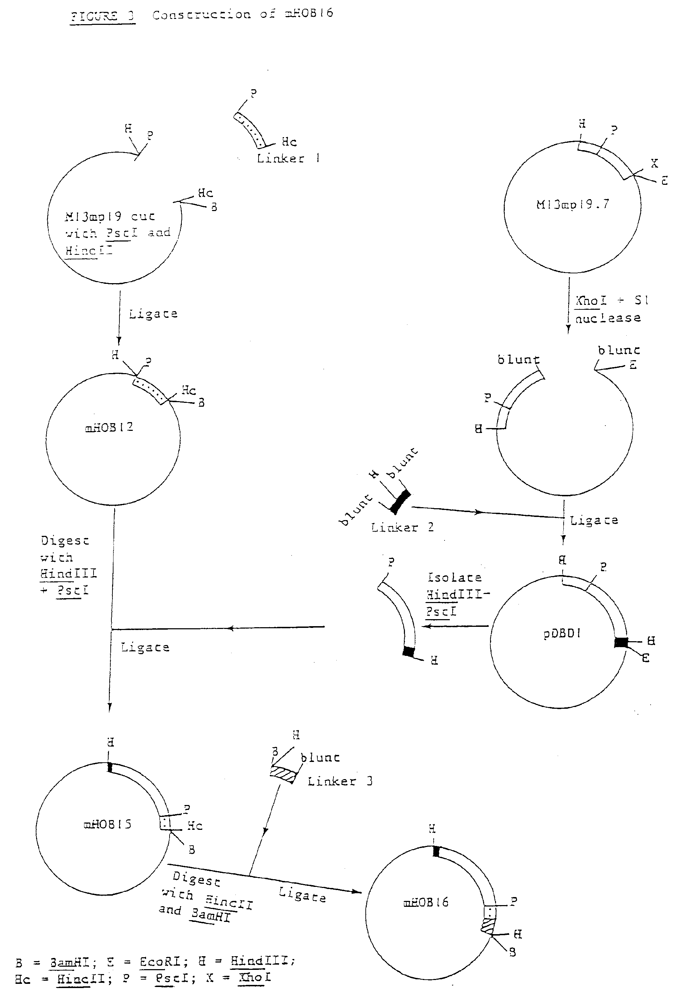

Linker 1 was ligated into the vector M13mp19 (Norrander et al, 1983) which had been digested with PstI and HincII and the ligation mixture was used to transfect E.coli strain XL1-Blue (Stratagene Cloning Systems, San Diego, CA). Recombinant clones were identified by their failure to evolve a blue colour on medium containing the chromogenic indicator X-gal (5-bromo-4-chloro-3-indolyl-β-D-galactoside) in the present of IPTG (isopropylthio-β-galactoside). DNA sequence analysis of template DNA prepared from bacteriophage particles of recombinant clones identified a molecule with the required DNA sequence, designated mHOB12 (Figure 3).

M13mp19.7 consists of the coding region of mature HSA in M13mp19 (Norrander et al, 1983) such that the codon for the first amino acid of HSA, GAT, overlaps a unique XhoI site thus: 
Asp Ala
5′ C T C G A G A T G C A 3′
3′ G A G C T C T A C G T 5′
XhoI
(EP-A-210 239). M13mp19.7 was digested with XhoI and made flush-ended by S1-nuclease treatment and was then ligated with the following oligonucleotide (Linker 2):

### Linker 2

5′ T C T T T T A T C C A A G C T T G G A T A A A A G A 3′
3′ A G A A A A T A G G T T C G A A C C T A T T T T C T 5′
HindIII

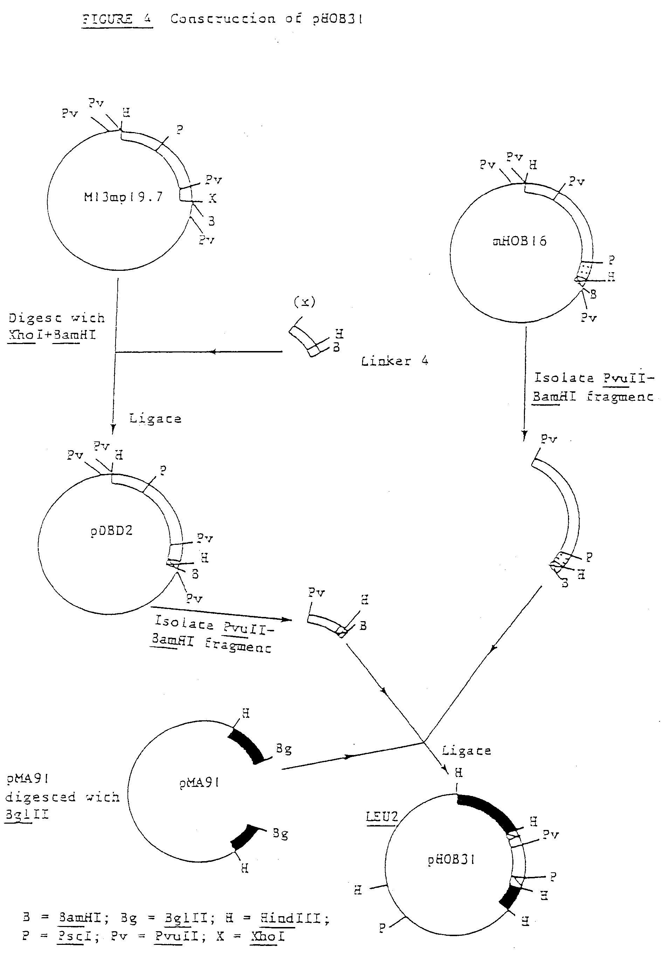

The ligation mix was then used to transfect E.coli XL1-Blue and template DNA was prepared from several plaques and then analysed by DNA sequencing to identify a clone, pDBD1 (Figure 4), with the correct sequence.

A 1.1 kb HindIII to PstI fragment representing the 5' end of the HSA coding region and one half of the inserted oligonucleotide linker was isolated from pDBD1 by agarose gel electrophoresis. This fragment was then ligated with double stranded mHOB12 previously digested with HindIII and PstI and the ligation mix was then used to transfect E.coli XL1-Blue. Single stranded template DNA was prepared from mature bacteriophage particles of several plaques. The DNA was made double stranded in vitro by extension from annealed sequencing primer with the Klenow fragment of DNA polymerase I in the presence of deoxynucleoside triphosphates. Restriction enzyme analysis of this DNA permitted the identification of a clone with the correct configuration, mHOB15 (Figure 4).

The following oligonucleotide (Linker 3) represents from the codon for the 382nd amino acid of mature HSA (glutamate, GAA) to the codon for lysine 389 which is followed by a stop codon (TAA) and a HindIII site and then a BamHI cohesive end:

### Linker 3

E E P Q N L I K J
5′ GAA GAG CCT CAG AAT TTA ATC AAA TAA GCTTG 3′
3′ CTT CTC GGA GTC TTA AAT TAG TTT ATT CGAACCTAG 5′

This was ligated into double stranded mHOB15, previously digested with HincII and BamHI. After ligation, the DNA was digested with HincII to destroy all non-recombinant molecules and then used to transfect E.coli XL1-Blue. Single stranded DNA was prepared from bacteriophage particles of a number of clones and subjected to DNA sequence analysis. One clone having the correct DNA sequence was designated mHOB16 (Figure 4).

A molecule in which the mature HSA coding region was fused to the HSA secretion signal was created by insertion of Linker 4 into BamHI and XhoI digested M13mp19.7 to form pDBD2 (Figure 4).

### Linker 4

M K W V S F
5′ GATCC ATG AAG TGG GTA AGC TTT
G TAC TTC ACC CAT TCG AAA

I S L L F L F S
ATT TCC CTT CTT TTT CTC TTT AGC
TAA AGG GAA GAA AAA GAG AAA TCG

S A Y S R G V F
TCG GCT TAT TCC AGG GGT GTG TTT
AGC CGA ATA AGG TCC CCA CAC AAA

R R
CG 3′
GCAGCT 5′

In this linker the codon for the fourth amino acid after the initial methionine, ACC for threonine in the HSA pre-pro leader sequence (Lawn et al, 1981), has been changed to AGC for serine to create a HindIII site.

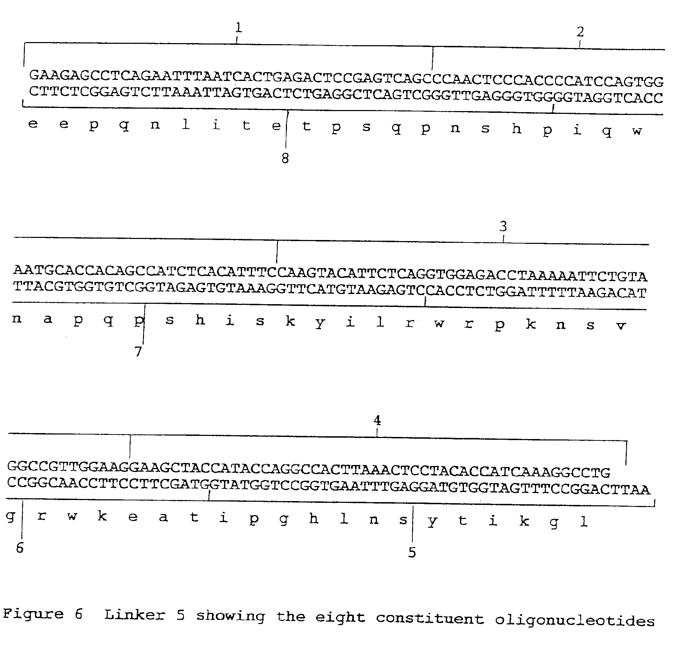

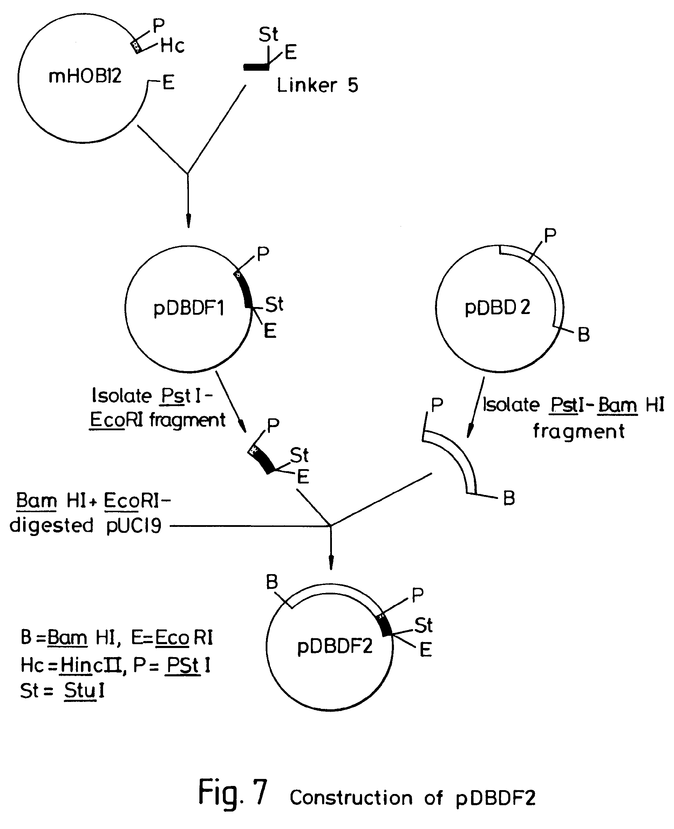

A sequence of synthetic DNA representing a part of the known HSA coding sequence (Lawn et al., 1981) (amino acids 382 to 387, Fig. 2), fused to part of the known fibronectin coding sequence (Kornblihtt et al., 1985) (amino acids 585 to 640, Fig. 2), was prepared by synthesising six oligonucleotides (Linker 5, Fig. 6). The oligonucleotides 2, 3, 4, 6, 7 and 8 were phosphorylated using T4 polynucleotide kinase and then the oligonucleotides were annealed under standard conditions in pairs, i.e. 1+8, 2+7, 3+6 and 4+5. The annealed oligonucleotides were then mixed together and ligated with mHOB12 which had previously been digested with the restriction enzymes HincII and EcoRI. The ligation mixture was then used to transfect E.coli XL1-Blue (Stratagene Cloning Systems, San Diego, CA). Single stranded template DNA was then prepared from mature bacteriophage particles derived from several independent plaques and then was analysed by DNA sequencing. A clone in which a linker of the expected sequence had been correctly inserted into the vector was designated pDBDF1 (Fig. 7). This plasmid was then digested with PstI and EcoRI and the approx. 0.24kb fragment was purified and then ligated with the 1.29kb BamHI-PstI fragment of pDBD2 (Fig. 7) and BamHI + EcoRI digested pUC19 (Yanisch-Perron, et al., 1985) to form pDBDF2 (Fig. 7).

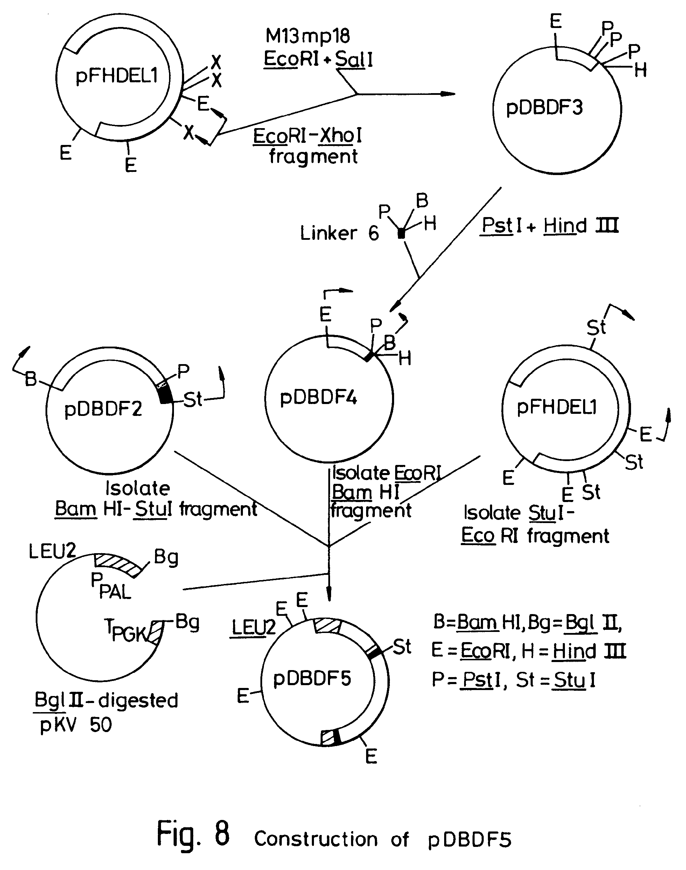

A plasmid containing a DNA sequence encoding full length human fibronectin, pFHDEL1, was digested with EcoRI and XhoI and a 0.77kb EcoRI-xhoI fragment (Fig. 8) was isolated and then ligated with EcoRI and salI digested M13 mp18 (Norrander et al., 1983) to form pDBDF3 (Fig. 8).

The following oligonucleotide linker (Linker 6) was synthesised, representing from the PstI site at 4784-4791 of the fibronectin sequence of EP-A-207 751 to the codon for tyrosine 1578 (Fig. 5) which is followed by a stop codon (TAA), a HindIII site and then a BamHI cohesive end:

### Linker 6

G P D Q T E M T I E G L
GGT CCA GAT CAA ACA GAA ATG ACT ATT GAA GGC TTG
A CGT CCA GGT CTA GTT TGT CTT TAC TGA TAA CTT CCG AAC

Q P T V E Y Stop
CAG CCC ACA GTG GAG TAT TAA GCTTG
GTC GGG TGT CAC CTC ATA ATT CGAACCTAG

This linker was then ligated with PstI and HindIII digested pDBDF3 to form pDBDF4 (Fig. 8). The following DNA fragments were then ligated together with BglII digested pKV50 (EP-A-258 067) as shown in Fig. 8: 0.68kb EcoRI-BamHI fragment of pDBDF4, 1.5kb BamHI-StuI fragment of pDBDF2 and the 2.2kb StuI-EcoRI fragment of pFHDEL1. The resultant plasmid pDBDF5 (Fig. 8) includes the promoter of EP-A-258 067 to direct the expression of the HSA secretion signal fused to DNA encoding amino acids 1-387 of mature HSA, in turn fused directly and in frame with DNA encoding amino acids 585-1578 of human fibronectin, after which translation would terminate at the stop codon TAA. This is then followed by the S.cerevisiae PGK gene transcription terminator. The plasmid also contains sequences which permit selection and maintenance in Escherichia coli and S.cerevisiae (EP-A-258 067).

This plasmid was introduced into S.cerevisiae S150-2B (leu2-3 leu2-112 ura3-52 trp1-289 his3- 1) by standard procedures (Beggs, 1978). Transformants were subsequently analysed and found to produce the HSA-fibronectin fusion protein.

### EXAMPLE 2 : HSA 1-195 FUSED TO Fn 585-1578

In this second example the first domain of human serum albumin (amino acids 1-195) is fused to amino acids 585-1578 of human fibronectin.

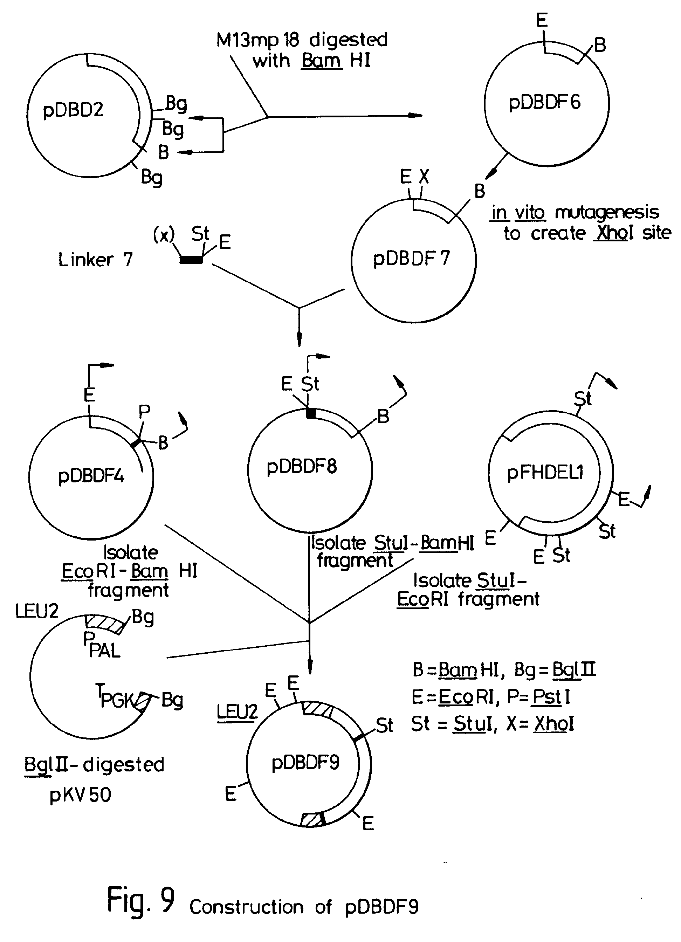

The plasmid pDBD2 was digested with BamHI and BglII and the 0.79kb fragment was purified and then ligated with BamHI-digested M13mp19 to form pDBDF6 (Fig. 6). The following oligonucleotide: 5'-C C A A A G C T C G A G G A A C T T C G-3'
was used as a mutagenic primer to create a XhoI site in pDBDF6 by in vitro mutagenesis using a kit supplied by Amersham International PLC. This site was created by changing base number 696 of HSA from a T to a G (Fig. 2). The plasmid thus formed was designated pDBDF7 (Fig. 9). The following linker was then synthesised to represent from this newly created XhoI site to the codon for lysine 195 of HSA (AAA) and then from the codon for isoleucine 585 of fibronectin to the ends of oligonucleotides 1 and 8 shown in Fig. 6.

### Linker 7

D E L R D E G K A S S A K
TC GAT GAA CTT CGG GAT GAA GGG AAG GCT TCG TCT GCC AAA
A CTT GAA GCC CTA CTT CCC TTC CGA AGC AGA CGG TTT

I T E T P S Q P N S H
ATC ACT GAG ACT CCG AGT CAG C
TAG TGA CTC TGA GGC TCA GTC GGG TTG AGG GTG G

This linker was ligated with the annealed oligonucleotides shown in Fig. 3, i.e. 2+7, 3+6 and 4+5 together with XhoI and EcoRI digested pDBDF7 to form pDBDF8 (Fig. 9). Note that in order to recreate the original HSA DNA sequence, and hence amino acid sequence, insertion of linker 7 and the other oligonucleotides into pDBDF7 does not recreate the XhoI site.

The 0.83kb BamHi-StuI fragment of pDBDF8 was purified and then was ligated with the 0.68kb EcoRI-BamHI fragment of pDBDF2 and the 2.22kb StuI-EcoRI fragment of pFHDEL1 into BglII-digested pKV50 to form pDBDF9 (Fig. 9). This plasmid is similar to pDBDF5 except that it specifies only residues 1-195 of HSA rather than 1-387 as in pDBDF5.

When introduced into S.cerevisiae S150-2B as above, the plasmid directed the expression and secretion of a hybrid molecule composed of residues 1-195 of HSA fused to residues 585-1578 of fibronectin.

### EXAMPLE 3 : HSA 1-387 FUSED TO Fn 585-1578, AS CLEAVABLE MOLECULE

In order to facilitate production of large amounts of residues 585-1578 of fibronectin, a construct was made in which DNA encoding residues 1-387 of HSA was separated from DNA encoding residues 585-1578 of fibronectin by the sequence 
I E G R
ATT GAA GGT AGA
TAA CTT CCA TCT
which specifies the cleavage recognition site for the blood clotting Factor X. Consequently the purified secreted product can be treated with Factor X and then the fibronectin part of the molecule can be separated from the HSA part.

To do this two oligonucleotides were synthesised and then annealed to form Linker 8.

### Linker 8

E E P Q N L I E G
GAA GAG CCT CAG AAT TTA ATT GAA GGT
CTT CTC GGA GTC TTA AAT TAA CTT CCA

R I T E T P S Q P
AGA ATC ACT GAG ACT CCG AGT CAG C
TCT TAG TGA CTC TGA GGC TCA GTC GGG

N S H

TTG AGG GTG G

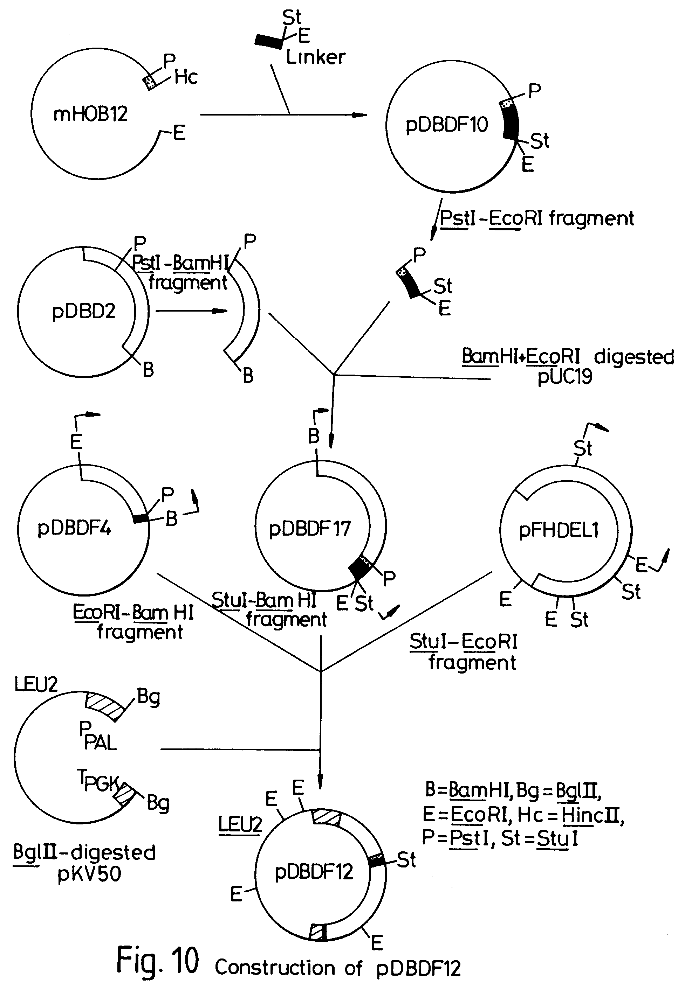

This linker was then ligated with the annealed oligonucleotides shown in Fig. 6, i.e. 2+7, 3+6 and 4+5 into HincII andEcoRI digested mHOB12, to form pDBDF10 (Fig. 7). The plasmid was then digested with PstI and EcoRI and the roughly 0.24kb fragment was purified and then ligated with the 1.29kb BamHI-PstI fragment of pDBD2 and BamHI and EcoRI digested pUC19 to form pDBDF11 (Fig. 10).

The 1.5kb BamHI-StuI fragment of pDBDF11 was then ligated with the 0.68kb EcoRI-BamH1 fragment of pDBDF4 and the 2.22kb StuI-EcoRI fragment of pFHDEL1 into BglII-digested pKV50 to form pDBDF12 (Fig. 10). This plasmid was then introduced into S.cerevisiae S150-2B. The purified secreted fusion protein was treated with Factor X to liberate the fibronectin fragment representing residues 585-1578 of the native molecule.

### REFERENCES

Beggs, J.D. (1978) Nature 275, 104-109

Kornblihtt et al. (1985) EMBO J. 4, 1755-1759

Lawn, R.M. et al. (1981) Nucl. Acid. Res. 9, 6103-6114

Maniatis, T. et al. (1982) Molecular cloning: A laboratory manual. Cold Spring Harbor Laboratory, Cold Spring Harbor, New York.

Messing, J. (1983) Methods Enzymol. 101, 20-78

Norrander, J. et al. (1983) Gene 26, 101-106

Sanger, F. et al. (1977) Proc. Natl. Acad. Sci. USA 74, 5463-5467

Yanisch-Perron, C. (1985) Gene 33, 103-119

## Claims (Claims for the following Contracting State(s): AT, BE, CH, LI, DE, DK, FR, IT, LU, NL, SE)

1. A fusion polypeptide comprising, as at least part of the N-terminal portion thereof, an N-terminal portion of HSA or a variant thereof and, as at least part of the C-terminal portion thereof, another polypeptide except that, when the said N-terminal portion of HSA is the 1-n portion where n is 369 to 419 or a variant thereof then the said polypeptide is (a) the 585 to 1578 portion of human fibronectin or a variant thereof, (b) the 1 to 368 portion of CD4 or a variant thereof, (c) platelet derived growth factor or a variant thereof, (d) transforming growth factor β or a variant thereof, (e) the 1-261 portion of mature human plasma fibronectin or a variant thereof, (f) the 278-578 portion of mature human plasma fibronectin or a variant thereof, (g) the 1-272 portion of mature human von Willebrand's Factor or a variant thereof, or (h) alpha-1-antitrypsin or a variant thereof.

2. A fusion polypeptide according to Claim 1 additionally comprising at least one N-terminal amino acid extending beyond the portion corresponding to the N-terminal portion of HSA.

3. A fusion polypeptide according to Claim 1 or 2 wherein there is a cleavable region at the junction of the said N-terminal or C-terminal portions.

4. A fusion polypeptide according to any one of the preceding claims wherein the said C-terminal portion is the 585 to 1578 portion of human plasma fibronectin or a variant thereof.

5. A transformed or transfected host having a nucleotide sequence so arranged as to express a fusion polypeptide according to any one of the preceding claims.

6. A process for preparing a fusion polypeptide by cultivation of a host according to Claim 5, followed by separation of the fusion polypeptide in a useful form.

7. A fusion polypeptide according to any one of Claims 1 to 4 for use in therapy.

## Claims (Claims for the following Contracting State(s): ES, GR)

1. A process for preparing a fusion polypeptide by (i) cultivation of a transformed or transfected host having a nucleotide sequence so arranged as to express a fusion polypeptide, followed by (ii) separation of the fusion polypeptide in a useful form, characterised in that the fusion polypeptide comprises as at least part of the N-terminal portion thereof, an N-terminal portion of HSA or a variant thereof and, as at least part of the C-terminal portion thereof, another polypeptide except that, when the said N-terminal portion of HSA is the 1-n portion where n is 369 to 419 or a variant thereof then the said polypeptide is (a) the 585 to 1578 portion of human fibronectin or a variant thereof, (b) the 1 to 368 portion of CD4 or a variant thereof, (c) platelet derived growth factor or a variant thereof, (d) transforming growth factor β or a variant thereof, (e) the 1-261 portion of mature human plasma fibronectin or a variant thereof, (f) the 278-578 portion of mature human plasma fibronectin or a variant thereof, (g) the 1-272 portion of mature human von Willebrand's Factor or a variant thereof, or (h) alpha-1-antitrypsin or a variant thereof.

2. A process according to Claim 1, wherein the fusion polypeptide additionally comprising at least one N-terminal amino acid extending beyond the portion corresponding to the N-terminal portion of HSA.

3. A process according to Claim 1 or 2 wherein, in the fusion polypeptide, there is a cleavable region at the junction of the said N-terminal or C-terminal portions.

4. A process according to any one of the preceding claims wherein the said C-terminal portion is the 585 to 1578 portion of human plasma fibronectin or a variant thereof.

## Patentansprüche (Patentansprüche für folgende(n) Vertragsstaat(en): AT, BE, CH, DE, DK, FR, IT, LU, NL, SE)

1. Fusionspolypeptid, umfassend als mindestens einen Teil seines N-terminalen Teils einen N-terminalen Teil von HSA oder eine Variante davon und als mindestens einen Teil seines C-terminalen Teils ein weiteres Polypeptid mit der Ausnahme, daß wenn es sich bei dem N-terminalen Teil von HSA um den Teil 1-n mit n = 369 bis 419 oder eine Variante davon handelt, das Polypeptid aus
(a) dem Teil 585 bis 1578 von Humanfibronectin oder einer Variante davon,
(b) dem Teil 1 bis 368 von CD4 oder einer Variante davon,
(c) dem "Platelet Derived Growth Factor" (PDGF) oder einer Variante davon,
(d) dem "Transforming Growth Factor β" (TGF β) oder einer Variante davon,
(e) dem Teil 1-261 von reifem Humanplasmafibronectin oder einer Variante davon,
(f) dem Teil 278-578 von reifem Humanplasmafibronectin oder einer Variante davon,
(g) dem Teil 1-272 von reifem Human-von Willebrand's-Faktor oder einer Variante davon oder
(h) Alpha-1-Antitrypsin oder einer Variante davon, besteht.

2. Fusionspolypeptid nach Anspruch 1, zusätzlich umfassend mindestens eine N-terminale Aminosäure, die länger als der dem N-terminalen Teil von HSA entsprechende Teil ist.

3. Fusionspolypeptid nach Anspruch 1 oder 2, bei dem sich an der Verbindung der N-terminalen oder C-terminalen Teile eine spaltbare Region befindet.

4. Fusionspolypeptid nach einem der vorhergehenden Ansprüche, wobei der C-terminale Teil aus dem Teil 585 bis 1578 von Humanplasmafibronectin oder einer Variante davon besteht.

5. Transformierter oder transfizierter Wirt mit einer Nukleotidsequenz, die so angeordnet ist, daß sie ein Fusionspolypeptid nach einem der vorhergehenden Ansprüche exprimieren kann.

6. Verfahren zur Herstellung eines Fusionspolypeptids durch Kultivieren eines Wirts nach Anspruch 5 und anschließendes Abtrennen des Fusionspolypeptids in einer geeigneten Form.

7. Fusionspolypeptid nach einem der Ansprüche 1 bis 4 zur therapeutischen Verwendung.

## Patentansprüche (Patentansprüche für folgende(n) Vertragsstaat(en): ES, GR)

1. Verfahren zur Herstellung eines Fusionspolypeptids durch
(i) Kultivieren eines transformierten oder transfektierten Wirts mit einer Nukleotidsequenz, die so angeordnet ist, daß sie ein Fusionspolypeptid exprimiert, und
(ii) anschließendes Abtrennen des Fusionspolypeptids in einer geeigneten Form,
dadurch gekennzeichnet, daß das Fusionspolypeptid als mindestens einen Teil seines N-terminalen Teils einen N-terminalen Teil von HSA oder eine Variante davon und als mindestens einen Teil seines C-terminalen Teils ein weiteres Polypeptid umfaßt, mit der Ausnahme, daß wenn es sich bei dem N-terminalen Teil von HSA um den Teil 1-n mit n= 369 bis 419 oder eine Variante davon handelt, das Polypeptid aus
(a) dem Teil 585-1578 von Humanfibronectin oder einer Variante davon,
(b) dem Teil 1-368 von CD4 oder einer Variante davon,
(c) dem Platelet Derived Growth Factor oder einer Variante davon,
(d) dem Transforming Growth Factor β oder einer Variante davon,
(e) dem Teil 1-261 von reifem Humanplasmafibronectin oder einer Variante davon,
(f) dem Teil 278-578 von reifem Humanplasmafibronectin oder einer Variante davon,
(g) dem Teil 1-272 von reifem Human-von Willebrand's-Faktor oder einer Variante davon oder
(h) α-1-Antitrypsin oder einer Variante davon besteht.

2. Verfahren nach Anspruch 1, wobei das Fusionspolypeptid zusätzlich mindestens eine N-terminale Aminosäure, die länger als der dem N-terminalen Teil von HSA entsprechende Teil ist, umfaßt.

3. Verfahren nach Anspruch 1 oder 2, wobei sich in dem Fusionspolypeptid an der Verbindung der N-terminalen oder C-terminalen Teile eine spaltbare Region befindet.

4. Verfahren nach einem der vorhergehenden Ansprüche, wobei der C-terminale Teil aus dem Teil 585-1578 von Humanplasmafibronectin oder einer Variante davon besteht.

## Revendications (Revendications pour l'(les) Etat(s) contractant(s) suivant(s): AT, BE, CH, DE, DK, FR, IT, LU, NL, SE)

1. Polypeptide fusionné comprenant en tant qu'au moins une partie de sa portion N-terminale, une portion N-terminale de HSA ou d'un variant de celle-ci et, en tant qu'au moins une partie de sa portion C-terminale, un autre polypeptide sauf que, lorsque cette portion N-terminale de HSA est la portion 1-n dans laquelle n est 369 à 419 ou un variant de celle-ci, ce polypeptide est (a) la portion 585 à 1578 de la fibronectine humaine ou un variant de celle-ci, (b) la portion 1 à 368 de CD4 ou un variant de celle-ci, (c) le facteur de croissance dérivé des plaquettes sanguines ou un variant de celui-ci, (d) le facteur de croissance β de transformation ou un variant de celui-ci, (e) la portion 1-261 de la fibronectine mature de plasma humain ou un variant de celle-ci, (f) la portion 278-578 de la fibronectine mature de plasma humain ou un variant de celle-ci, (g) la portion 1-272 du facteur humain mature de von Willebrand ou un variant de celle-ci, ou (h) l'alpha-1-antitrypsine ou un variant de celle-ci.

2. Polypeptide fusionné suivant la revendication 1, comprenant de plus au moins un acide aminé N-terminal se prolongeant au-delà de la portion correspondant à la portion N-terminale de HSA.

3. Polypeptide fusionné suivant les revendications 1 ou 2, dans lequel il y a une région susceptible d'être coupée à la jonction de ces portions N-terminale et C-terminale.

4. Polypeptide fusionné suivant l'une quelconque des revendications précédentes, dans lequel cette portion C-terminale est la portion 585 à 1578 de la fibronectine de plasma humain ou un variant de celle-ci.

5. Hôte transformé ou transfecté ayant une séquence de nucléotides arrangée de façon à exprimer un polypeptide fusionné suivant l'une quelconque des revendications précédentes.

6. Procédé pour préparer un polypeptide fusionné par culture d'un hôte suivant la revendication 5, suivie de la séparation du polypeptide fusionné sous une forme utile.

7. Polypeptide fusionné suivant l'une quelconque des revendications 1 à 4 utilisable en thérapie.

## Revendications (Revendications pour l'(les) Etat(s) contractant(s) suivant(s): ES, GR)

1. Procédé pour préparer un polypeptide fusionné par (i) la culture d'un hôte transformé ou transfecté ayant une séquence de nucléotides arrangée de façon à exprimer un polypeptide fusionné, suivie de (ii) la séparation du polypeptide fusionné sous une forme utilie, caractérisé en ce que le polypeptide fusionné comprend, en tant qu'au moins une partie de sa portion N-terminale, une portion N-terminale de HSA ou d'un variant de celle-ci et, en tant qu'au moins une partie de sa portion C-terminale, un autre polypeptide sauf que, lorsque cette portion N-terminale de HSA est la portion 1-n dans laquelle n est 369 à 419 ou un variant de celle-ci, ce polypeptide est alors (a) la portion 585 à 1578 de la fibronectine humaine ou un variant de celle-ci, (b) la portion 1 à 368 de CD4 ou un variant de celle-ci, (c) le facteur de croissance dérivé des plaquettes sanguines ou un variant de celui-ci, (d) le facteur de croissance β de transformation ou un variant de celui-ci, (e) la portion 1-261 de la fibronectine mature de plasma humain ou un variant de celle-ci, (f) la portion 278-578 de la fibronectine mature de plasma humain ou un variant de celle-ci, (g) la portion 1-272 du facteur humain mature de von Willebrand ou un variant de celle-ci, ou (h) l'alpha-1-antitrypsine ou un variant de celle-ci.

2. Procédé suivant la revendication 1, dans lequel le polypeptide fusionné comprend de plus au moins un acide aminé N-terminal se prolongeant au-delà de la portion correspondant à la portion N-terminale de HSA.

3. Procédé suivant les revendications 1 ou 2 dans lequel, dans le polypeptide fusionné, il y a une région susceptible d'être coupée à la jonction de ces portions N-terminale et C-terminale.

4. Procédé suivant l'une quelconque des revendications précédentes, dans lequel cette portion C-terminale est la portion 585 à 1578 de la fibronectine de plasma humain ou un variant de celle-ci.
